(19) Europäisches Patentamt
European Patent Office
Office européen des brevets

(11) Veröffentlichungsnummer: **0 027 941**
**B1**

(12) # EUROPÄISCHE PATENTSCHRIFT

(45) Veröffentlichungstag der Patentschrift: **08.12.82**

(51) Int. Cl.[5]: **C 07 C 51/60**, C 07 C 53/40, C 07 C 53/48, C 07 C 57/66, C 07 C 63/10

(21) Anmeldenummer: **80106197.9**

(22) Anmeldetag: **11.10.80**

(54) **Verfahren zur Herstellung von Carbonsäurehalogeniden.**

(30) Priorität: **26.10.79 DE 2943432**

(43) Veröffentlichungstag der Anmeldung: **06.05.81 Patentblatt 81/18**

(45) Bekanntmachung des Hinweises auf die Patenterteilung: **08.12.82 Patentblatt 82/49**

(84) Benannte Vertragsstaaten: **BE CH DE FR GB IT LI**

(56) Entgegenhaltungen: **DE-A-2 909 444**

(73) Patentinhaber: **BAYER AG, Zentralbereich Patente, Marken und Lizenzen, D-5090 Leverkusen 1, Bayerwerk (DE)**

(72) Erfinder: **Heydkamp, Wolfgang, Dr., Grüner Weg 18, D-5090 Leverkusen-1 (DE)**
Erfinder: **Rauchschwalbe, Günter, Dr., Hofstrasse 28, D-5000 Köln 80 (DE)**
Erfinder: **Trescher, Viktor, Dr., Völklinger Strasse 7, D-5090 Leverkusen (DE)**
Erfinder: **Steffan, Guido, Dr., Im Herzogenfeld 52, D-5068 Odenthal (DE)**

EP 0 027 941 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Verfahren zur Herstellung von Carbonsäurehalogeniden

Die Erfindung betrifft ein Verfahren zur Herstellung von Carbonsäurehalogeniden aus den zugehörigen Carbonsäuren und Thionylhalogenid in Gegenwart einer gegebenenfalls substituierten Harnstoff-Verbindung.

Es ist bekannt, daß organische Carbonsäuren mit Thionylchlorid unter Bildung von Carbonsäurechloriden reagieren (Organikum, 5. Auflage, Seite 408, VEB deutscher Verlag der Wissenschaften, 1965). Die Verwendung von Thionylchlorid ist hierbei gegenüber der Verwendung von Phosphorchloriden besonders günstig, da als Reaktionsprodukte des Thionylchlorids gasförmiges $SO_2$ und HCl entstehen, die leicht vom Säurechlorid abgetrennt werden können. Im Vergleich zum grundsätzlich ebenfalls verwendbaren Phosgen ist Thionylchlorid erheblich weniger toxisch und kann daher ohne aufwendige Sicherheitsmaßnahmen eingesetzt werden.

Bei der Anwendung im industriellen Maßstab ist es wünschenswert, diese Reaktion, die im allgemeinen auch ohne Zusätze abläuft, durch den Zusatz katalytisch wirksamer Substanzen zu beschleunigen. In manchen Fällen läuft die gesamte Reaktion sogar so langsam ab, daß eine Durchführung ohne beschleunigende Zusätze überhaupt nicht in Frage kommt. Dies ist beispielsweise bei der Umsetzung von Trichloressigsäure der Fall (J. Am. Chem. Soc. 50, 145 (1928)).

Als reaktionsbeschleunigender Zusatz ist N,N'-Dimethylformamid empfohlen worden (Schweizer Patent 333 502; Helv. Chim. Acta 42, 1653 (1959)). Es ist jedoch inzwischen bekannt geworden, daß aus Dimethylformamid und Thionylchlorid N,N'-Dimethyl-carbamoylchlorid entsteht (C. A. 75, 48 340 m), das an Mäusen krebserregend wirkt (C. A. 77, 97 540 b). Die Verwendung von Dimethylformamid als Katalysator bei der Herstellung von Säurechloriden mit Hilfe von Thionylchlorid ist deshalb nur unter aufwendigen technischen Sicherheitsmaßnahmen vertretbar.

Als reaktionsbeschleunigender Zusatz wurde auch Pyridin empfohlen (J. Org. Chem. 6, 852 (1941)). Die Verwendung von Pyridin kann aber Nachteile mit sich bringen, da das aus Pyridin und Chlorwasserstoff entstehende Pyridin-hydrochlorid bei erhöhter Temperatur sublimieren und damit zu Verstopfungen der Apparatur führen kann.

Weiterhin ist die Verwendung von Tetramethylharnstoff als katalytisch wirkende Substanz bei der Herstellung von Carbonsäurechloriden bekannt (Russisches Patent 223 087; zitiert nach C. A. 70, 19 607 v (1969)). Der hohe Preis des Tetramethylharnstoffs steht jedoch seiner Verwendung im technischen Maßstab entgegen. Weiterhin ist die Verwendung von Tetramethylharnstoff nicht unbedenklich, da er teratogene Wirkung zeigen kann, die beispielsweise N,N'-Dimethylharnstoff nicht zeigt (C. A. 78, 39 056 h; C. A. 71, 79 274 q). Darüber hinaus kann auch aus Tetramethylharnstoff und Thionylchlorid Dimethylcarbamoylchlorid entstehen, so daß gegen die Verwendung von Tetramethylharnstoff Bedenken bestehen.

Andererseits ist jedoch zu erwarten, daß Harnstoff oder physiologisch unbedenkliche Harnstoff-Verbindungen, die an den Stickstoffatomen nicht oder nicht vollständig substituiert sind, mit Carbonsäurechloriden zu Ureiden reagieren (Beyer, Lehrbuch der organischen Chemie, 9. Auflage, 1962, Seite 266), wodurch einerseits die Ausbeute an Säurechlorid gesenkt und andererseits auch schon nach mäßigem Umsatz der Katalysator verbraucht würde. So wird beispielsweise nach der US-Patentschrift 4 129 595 bei der Reaktion von Glykolsäure mit Thionylchlorid in Gegenwart von Tetramethylharnstoff eine Ausbeute von mehr als 95% erreicht, während in Anwesenheit von nicht substituiertem Harnstoff die Ausbeute auf 48% abfällt.

Es wurde nun ein Verfahren zur Herstellung von Carbonsäurehalogeniden durch Umsetzung einer Carbonsäure der Formel

$$R-COOH \qquad (I)$$

in der

R für Aryl oder $R^1R^2R^3C-$ steht,

worin

$R^1$, $R^2$ und $R^3$ unabhängig voneinander Wasserstoff, Halogen, Alkyl oder Alkenyl bedeuten und worin weiterhin zwei der Reste $R^1$ bis $R^3$ gemeinsam eine Alkylengruppe oder eine über eine Doppelbindung gebundene Alkylidengruppe bedeuten können, wobei die Kohlenstoff enthaltenden Reste durch Carboxyl, eine Estergruppe, deren Alkoholkomponente 1 bis 4 Kohlenstoffatome enthält, $C_1-C_{10}$-Alkoxy, Aryloxy, Aralkoxy, eine $C_1-C_{10}$-Thioethergruppe, Niederalkyl, Cyano, Nitro oder Halogen substituiert sein können,

mit einem Thionylhalogenid der Formel

$$SOX_2 \qquad (II)$$

in der

X  für Chlor oder Brom steht,

in Gegenwart von Zusätzen gefunden, das dadurch gekennzeichnet ist, daß man als Zusatz 0,01 bis 5 Mol-%, bezogen auf die Äquivalente der eingesetzten Carbonsäure, einen gegebenenfalls substituierten Harnstoff-Verbindung der Formel

$$R^4NH-CY-NR^5R^6 \qquad (III)$$

in der

Y  doppelt gebundenen Sauerstoff, Schwefel oder gegebenenfalls substituierten Stickstoff, $R^7-N=$ bedeutet und

$R^4$, $R^5$, $R^6$ und $R^7$ unabhängig voneinander für Wasserstoff, Alkyl, Aralkyl, Aryl oder Acyl stehen, und wobei weiterhin die zwei Reste $R^4$ und $R^5$, $R^5$ und $R^6$ oder $R^5$ und $R^7$ eine Alkylengruppe bilden können und wobei die Kohlenstoff enthaltenden Reste durch die obengenannten Substituenten substituiert sein können,

in freier Form oder in Form ihres Salzes, gegebenenfalls in Gegenwart eines inerten Lösungsmittels, einsetzt.

Als Alkyl seien beispielsweise geradkettige oder verzweigte Kohlenwasserstoffreste mit 1 bis 20, bevorzugt 1 bis 10, besonders bevorzugt 1 bis 4 Kohlenstoffatomen genannt, wie Methyl, Ethyl, Propyl, Isopropyl, Butyl, Isobutyl, Hexyl, Decyl, Dodecyl, Stearyl oder Eikosyl. In ganz besonders bevorzugter Weise seien Methyl und Ethyl genannt. Als Alkyl seien weiterhin auch gesättigte carbocyclische oder bicyclische Reste genannt, wie Cyclopropyl, Cyclobutyl, Cyclopentyl, Cyclohexyl oder Decahydronaphthyl.

Als Alkenyl seinen beispielsweise geradkettige oder verzweigte ungesättigte Reste mit 2 bis 20, bevorzugt 2 bis 10, besonders bevorzugt 2 bis 4 Kohlenstoffatomen genannt, wie Vinyl, 1-Propenyl, 2-Propenyl, 2-Butenyl, 2-Methyl-1-propenyl, Hexenyl, Octenyl, Decenyl, Dodecenyl, Oleyl oder Eikosenyl.

Als Aralkyl seien beispielsweise Kohlenwasserstoffreste mit bis zu 2 Kohlenstoffatomen im aliphatischen Teil und bis zu 14 Kohlenstoffatomen im aromatischen Teil genannt, wie Benzyl, Phenylethyl, Naphthylmethyl, Naphthylethyl, Anthrylmethyl oder Anthrylethyl. Bevorzugtes Aralkyl ist Benzyl.

Als Aryl seinen beispielsweise aromatische carbocyclische Reste mit 6 bis 14 Kohlenstoffatomen genannt, wie Phenyl, Biphenyl, Naphthyl oder Anthryl, bevorzugt Phenyl oder Naphthyl, besonders bevorzugt Phenyl.

Für den Fall, daß zwei der Reste $R^1$ bis $R^3$ gemeinsam oder die zwei Reste $R^4$ und $R^5$, $R^5$ und $R^6$ oder $R^5$ und $R^7$ eine Alkylengruppe bilden können, sei eine solche von 2 bis 5 Kohlenstoffatomen genannt, wie Ethylen, Trimethylen, Tetramethylen oder Pentamethylen.

Für den Fall, daß zwei der Reste $R^1$ bis $R^3$ gemeinsam eine über eine Doppelbildung gebundene Alkylidengruppe bedeuten können, sei eine solche mit 1 bis 10, bevorzugt 1 bis 3 Kohlenstoffatomen genannt, wie Vinyliden ($CH_2=$), Ethyliden ($CH_3-CH=$), Propyliden, iso-Propyliden, Hexyliden oder Decyliden, bevorzugt Vinyliden, Ethyliden und iso-Propyliden.

Als Acyl seien beispielsweise genannt: Acetyl, Chloracetyl, Benzoyl, 4-Chlorbenzoyl.

Als Acyl kommen weiterhin Reste in Frage, die den genannten Carbonsäuren der Formel (I) zugrunde liegen, wobei es unerheblich ist, ob der Acylrest in der zum Halogenid umzusetzenden Carbonsäure mit dem Acylrest im N-Acylharnstoff übereinstimmt oder nicht.

Die genannten offenkettigen oder cyclischen, gesättigten und ungesättigten aliphatischen und aromatischen Reste können weiterhin durch eine Carboxylgruppe, eine Estergruppe, deren Alkoholkomponente 1 bis 4 Kohlenstoffatome enthält, eine Alkoxygruppe mit 1 bis 10, bevorzugt 1 bis 4, besonders bevorzugt 1 bis 2 Kohlenstoffatomen, eine Aryloxygruppe, bevorzugt Phenyloxygruppe, eine Aralkoxygruppe, bevorzugt Benzyloxygruppe, eine Thioethergruppe mit 1 bis 10, bevorzugt 1 bis 4, besonders bevorzugt 1 bis 2 Kohlenstoffatomen eine niedere Alkylgruppe, bevorzugt Methyl oder Ethyl, sowie durch Cyano, Nitro oder Halogen substituiert sein.

Als Halogen sei beispielsweise Fluor, Chlor, Brom oder Jod, bevorzugt Chlor oder Brom, genannt.

Für den Fall, daß Y doppelt gebundenen Sauerstoff darstellt, seien als Verbindungen der Formel (III) Harnstoff oder teilweise substituierte Harnstoffe genannt. Für den Fall, daß Y doppelt gebundenen Schwefel darstellt, seien als Verbindungen der Formel (III) Thioharnstoff oder teilweise substituierte Thioharnstoffe genannt. Für den Fall, daß Y doppelt gebundenen Stickstoff bedeutet, seien als Verbindungen der Formel (III) Guanidin und an den endständigen Stickstoffatomen teilweise substituiertes Guanidin, sowie die entsprechenden, am doppelt gebundenen Stickstoffatom Alkyl- oder Aryl-substituierten Guanidine, genannt.

Die Harnstoff-Verbindungen der Formel (III) können im erfindungsgemäßen Verfahren sowohl in

freier Form als auch in Form ihrer Salze, beispielsweise der Hydrochloride, der Hydrobromide, der Sulfate, der Phosphate oder der Carbonate, eingesetzt werden.

Als bevorzugte Carbonsäuren im Rahmen der Formel (I) für das erfindungsgemäße Verfahren seien solche der Formel

$$R^8-COOH \qquad (IV)$$

genannt, in der

$R^8$ für Phenyl oder $R^9R^{10}R^{11}C-$ steht,

worin

$R^9$ für Wasserstoff, Halogen oder $C_1-C_{10}$-Alkyl steht,
$R^{10}$ und $R^{11}$ unabhängig voneinander Wasserstoff oder Halogen bedeuten,

wobei Aryl und Alkyl ihrerseits durch Halogen, Nitro, Alkyl, Carboxyl, die Carboxylmethylestergruppe, die Carboxylethylestergruppe, $C_1-C_4$-Alkoxy oder Phenoxy substituiert sein können und wobei zwei der Reste $R^9$, $R^{10}$ und $R^{11}$ eine über eine Doppelbindung gebundene Alkylidengruppen bedeuten können.

Als Carbonsäuren im Rahmen der Formel (I) für das erfindungsgemäße Verfahren seien besonders bevorzugt solche der Formel

$$R^{12}-COOH \qquad (V)$$

genannt, in der

$R^{12}$ für Phenyl oder für $R^{13}R^{14}R^{15}C-$ steht,

worin

$R^{13}$ Wasserstoff, Chlor, Brom oder $C_1-C_4$-Alkyl bedeutet,
$R^{14}$ und $R^{15}$ unabhängig voneinander für Wasserstoff, Chlor oder Brom stehen,

wobei Phenyl und $C_1-C_4$-Alkyl ihrerseits durch Chlor, Brom, Nitro, Methoxy, Methyl, Ethyl oder Carboxyl substituiert sein können und wobei zwei der Rest $R^{13}$, $R^{14}$ und $R^{15}$ gemeinsam eine über eine Doppelbindung gebundene $C_1-C_3$-Alkylidengruppe bilden können.

Als Beispiele für Carbonsäuren für das erfindungsgemäße Verfahren seien genannt: Essigsäure, Propionsäure, Buttersäure, Isobuttersäure, Valeriansäure, iso-Valeriansäure, Trimethyl-essigsäure, Capronsäure, Caprylsäure, Caprinsäure, Laurinsäure, Palmitinsäure, Stearinsäure, Acrylsäure, Methacrylsäure, Crotonsäure, Isocrotonsäure, Vinylessigsäure, Ölsäure, Chloressigsäure, Dichloressigsäure, Trichloressigsäure, Bromessigsäure, Dibromessigsäure, Tribromessigsäure, $\alpha$-Chlorpropionsäure, $\alpha$-Chlorbuttersäure, Malonsäure, Bernsteinsäure, Malonsäuremonomethylester, Bernsteinsäuremonomethylester, Phenylessigsäure, Phenyloxyessigsäure, $\beta$-Phenylpropionsäure, $\beta$-Phenyloxypropionsäure, Cyclohexancarbonsäure, Cyclohexancarbonsäure, Undecylensäure, Methoxyessigsäure, Tiglinsäure, Angelikasäure, Fumarsäure, Maleinsäure, Brassylsäure, Benzoesäure, Naphthoesäure, Tolylsäure, 2-Chlorbenzoesäure, 4-Chlorbenzoesäure, 2-Nitrobenzoesäure, 4-Nitrobenzoesäure, 3-Cyanobenzoesäure, Isophthalsäure, Terepthalsäure, 3-Phenoxybenzoesäure, Terephthalsäure-monomethylester, 2-Methylthio-benzoesäure, 4-tert.-Butyl-benzoesäure, 2,4-Dichlorbenzoesäure. Die genannten Carbonsäuren sind dem Fachmann seit langem bekannt.

Als Harnstoff-Verbindungen im Rahmen der Formel (III) für das erfindungsgemäße Verfahren seien bevorzugt solche der Formel

$$R^{16}NH-CY^1-NH-R^{17} \qquad (VI)$$

genannt, in der

$Y^1$ doppelt gebundenen Sauerstoff, doppelt gebundenen Schwefel oder gegebenenfalls substituierten doppelt gebundenen Stickstoff $R^{18}-N=$ bedeutet und
$R^{16}$, $R^{17}$ und $R^{18}$ unabhängig voneinander für Wasserstoff, $C_1-C_4$-Alkyl, Phenyl oder Acyl stehen.

Die Harnstoff-Verbindungen der Formel (VI) können sowohl in freier Form als auch in Form ihrer Salze im erfindungsgemäßen Verfahren eingesetzt werden.

Als Harnstoff-Verbindung für das erfindungsgemäße Verfahren sei besonders bevorzugt der freie

nicht substituierte Harnstoff genannt.

Als Harnstoff-Verbindungen für das erfindungsgemäße Verfahren seien beispielsweise genannt: Harnstoff, N-Methylharnstoff, N,N'-Dimethylharnstoff, N,N'-Diphenylharnstoff, Thioharnstoff, N-Methylthioharnstoff, Guanidinhydrogencarbonat, N,N-Dimethylharnstoff, N,N-Diphenylharnstoff, N-Phenylharnstoff, N,N'-Dimethylthioharnstoff, N,N'-Diphenylthioharnstoff, N-Methyl-N'-phenylharnstoff, Trimethylharnstoff, N,N'-Dimethyl-N-phenylharnstoff, Acetylharnstoff, Chloracetylharnstoff, Benzoylharnstoff, 4-Chlor-benzoyl-harnstoff, N,N'-Diacetylharnstoff, N,N'-Di-chloracetylharnstoff, N,N'-Dibenzoylharnstoff, N,N'-Di-4-chlorbenzoylharnstoff.

N-Acylharnstoff-Verbindungen können als solche eingesetzt werden oder aber unter den Reaktionsbedingungen vor oder während der Bildung der Säurehalogenide im Reaktionsansatz, hergestellt werden, z. B. aus Säurehalogenid und Harnstoff.

Die erfindungsgemäß einsetzbaren Harnstoff-Verbindungen sind dem Fachmann bekannt und können nach bekannten Verfahren hergestellt werden, beispielsweise Harnstoff oder teilweise substituierte Harnstoffe durch Erhitzen von gegebenenfalls substituiertem Ammoniumcyanat oder durch Umsetzung von Phosgen oder Isocyansäureestern mit Ammoniak oder Aminen, für Thioharnstoff oder teilweise substituierte Thioharnstoffe analog durch Erwärmen von gegebenenfalls substituiertem Ammoniumrhodanid oder durch Umsetzung von Thiophosgen mit Ammoniak oder Aminen und für Guanidin oder substituierte Guanidine durch Erhitzen von Cyanamid mit gegebenenfalls substituiertem Ammoniumchlorid oder aus Dicyandiamid und gegebenenfalls substituiertem Ammoniumnitrat (vgl. Lehrbücher der organischen Chemie).

Im erfindungsgemäßen Verfahren ist es ausreichend die Harnstoff-Verbindung in kleiner Menge zuzusetzen. Als solche sei eine Menge von 0,01 bis 5 Mol-%, bevorzugt 0,02 bis 2 Mol-%, besonders bevorzugt 0,03 bis 1 Mol-%, bezogen auf die Äquivalente der eingesetzten Carbonsäure, genannt.

Als Thionylhalogenid der Formel (II) sei beispielsweise Thionylchlorid oder Thionylbromid, bevorzugt Thionylchlorid, genannt.

Das Thionylhalogenid wird zur Erzielung eines vollständigen Umsatzes in mindestens äquivalenter Menge, bezogen auf die freien Carboxyläquivalente, eingesetzt. Beispielsweise sei eine Menge von 1 bis 3 Mol, bevorzugt 1,1 bis 2 Mol, besonders bevorzugt 1,2 bis 1,6 Mol pro Carbonsäureäquivalent genannt.

Als Temperatur für das erfindungsgemäße Verfahren, bei der die Reaktion vervollständigt wird, sei beispielsweise eine solche von 0° bis 150° C, bevorzugt 20 bis 150° C, besonders bevorzugt 50 bis 100° C, genannt. In vielen Fällen, besonders bei größeren Mengen der Reaktionspartner, kann es vorteilhaft sein, zur Beherrschung der Wärmetönung der Reaktion und zur Beherrschung der Abspaltung flüchtiger Stoffe in der Reaktion die Reaktionsführung bei einer tieferen Temperatur, beispielsweise bei Raumtemperatur, zu beginnen und planmäßig, entsprechend der Gasentwicklung, auf die gewählte Endtemperatur, die oben beschrieben wurde, zu steigern.

Die zur Vervollständigung der Reaktion erforderliche Höchsttemperatur im Rahmen des genannten Temperaturbereiches ist selbstverständlich etwas von der Reaktivität der eingesetzten Carbonsäure abhängig.

In besonders bevorzugter Weise wird die Reaktion bei der Rückflußtemperatur der Reaktionsmischung vervollständigt. Diese Temperatur ist in vielen Fällen die Siedetemperatur des bevorzugt eingesetzten Thionylchlorids. Für den Fall, daß der molare Überschuß des Thionylchlorids sehr gering ist, kann gegen Ende der Reaktion auch ohne Anwendung weiterer Maßnahmen die Temperatur des Reaktionsgemisches in Folge einer leichten Überhitzung über der Siedetemperatur des Thionylchlorids liegen.

Der Druck über dem Reaktionsgemisch ist für die Durchführung des erfindungsgemäßen Verfahrens unkritisch, so daß aus Gründen der apparativen Vereinfachung im allgemeinen unter Normaldruck gearbeitet wird. Jedoch kann zur Beeinflussung der Höhe der Rückflußtemperatur des Reaktionsgemisches auch unter einem erhöhten Druck, beispielsweise bis zu 10 bar, gearbeitet werden.

Das erfindungsgemäße Verfahren kann absatzweise oder kontinuierlich durchgeführt werden. Bei kontinuierlicher Reaktionsführung kann beispielsweise in einer dem Fachmann bekannten Weise in einem Umlaufreaktor gearbeitet werden, bei dem an einer Stelle des Stoffumlaufs durch Einzeleinlässe oder einen gemeinsamen Einlaß die Carbonsäure, das Thionylhalogenid und die Harnstoff-Verbindung zugeführt werden, während, in Fließrichtung der Reaktionsmischung gesehen, vor dem Wiedererreichen dieser Zuführungsstellen ein Teil des Reaktionsgemisches dem Umlaufreaktor entnommen wird und in üblicher Weise in seine Komponenten aufgetrennt wird. Der bei der Auftrennung des Reaktionsgemisches anfallende Rückstand, der die eingesetzte Harnstoff-Verbindung enthält, oder die separat isolierte Harnstoff-Verbindung kann im erfindungsgemäßen Verfahren erneut eingesetzt werden.

Das erfindungsgemäße Verfahren kann lösungsmittelfrei oder in Gegenwart eines unter den Reaktionsbedingungen inerten Lösungsmittels durchgeführt werden. Die Gegenwart eines Lösungsmittels verkleinert zwar in Abhängigkeit von seiner angewendeten Menge die Raum-Zeit-Ausbeute, sie kann jedoch zur Abmilderung der Reaktion und/oder zur Einstellung einer bestimmten Temperatur des Reaktionsgemisches wünschenswert sein. Für den Fall, daß ein Lösungsmittel

angewendet wird, sei hierfür beispielsweise genannt: ein Kohlenwasserstoff, wie Pentan, Hexan, Haptan, Octan, Cyclohexan, Methylcyclohexan, Benzol, Toluol, Xylol, Ethylbenzol oder Gemische hiervon; ein aliphatischer oder aromatischer Halogenkohlenwasserstoff, wie Methylenchlorid, Chloroform, Dichlorethan, Trichlorethan, Tetrachlorethan, Dichlorethylen, Trichlorethylen, Tetrachlorethylen, Monochlorbenzol, Dichlorbenzol, Monochlortoluol, Dichlortoluol, Monochlorxylol, Dichlorxylol oder Gemische hiervon; ein Sulfoxid, wie Dimethysulfoxid; ein Sulfon, wie Tetramethylensulfon, ein Ester wie Methylacetat oder Ethylacetat; ein Nitril, wie Acetonitril.

Im erfindungsgemäßen Verfahren können Carbonsäurehalogenide der Formel

$$R-COX \qquad (VII)$$

hergestellt werden, in der
R und X die oben angegebene Bedeutung haben.

Das erfindungsgemäße Verfahren sei am Beispiel der Umsetzung von Monochloressigsäure mit Thionylchlorid in Gegenwart von nicht substituiertem Harnstoff durch die folgende Reaktionsgleichung erläutert:

$$Cl-CH_2-COOH + SOCl_2 \xrightarrow{[O=C(NH_2)_2]} Cl-CH_2-COCl + HCl + SO_2$$

Das Zusammengeben der Reaktionspartner kann in beliebiger Reihenfolge geschehen. Vielfach wird man die Carbonsäure und die Harnstoff-Verbindung vorlegen und das Thionylhalogenid bei Raumtemperatur ganz vorlegen oder im Verlaufe der Reaktion in einigen Portionen zum Reaktionsgemisch zufügen. Wenn die Reaktion nicht bereits bei Raumtemperatur einsetzt, wird das Gemisch vorsichtig erwärmt, bis die Reaktion einsetzt, was an der Entwicklung der Gase Chlorwasserstoff und Schwefeldioxid erkennbar ist. Die Reaktionstemperatur wird sodann entsprechend dem Fortschritt der Reaktion oder nach einem festen Temperaturprogramm so weit gesteigert, daß schließlich die Endtemperatur in dem weiter oben beschriebenen Temperaturintervall erreicht wird. Das Ende der Reaktion ist im allgemeinen an der beendeten Gasentwicklung zu erkennen. Das Gemisch kann sodann durch übliche Maßnahmen, beispielsweise durch Destillation in gegebenenfalls überschüssiges Thionylhalogenid, das gewünschte Carbonsäurehalogenid und einen Rückstand getrennt werden. Der Rückstand, der nicht umgesetzte Carbonsäure, gegebenenfalls deren Anhydrid, sowie die Harnstoff-Verbindung enthält, kann im allgemeinen dem nächsten Ansatz zugefügt werden.

Für viele Anwendungszwecke ist die Reinheit des Rohproduktes ausreichend, so daß auf eine Abtrennung des Zusatzes verzichtet und das rohe Reaktionsprodukt direkt weiter verarbeitet werden kann.

Der erfindungsgemäße Zusatz einer Harnstoff-Verbindung beschleunigt die Reaktion zwischen einer Carbonsäure und einem Thionylhalogenid, so daß einmal höhere Raum-Zeit-Ausbeuten gegenüber dem Stand der Technik erzielt werden können und zum anderen häufig bei niedrigeren Reaktionstemperaturen als beim Stand der Technik gearbeitet werden kann, wodurch die Bildung unerwünschter Nebenprodukte zurückgedrängt wird und Carbonsäurechloride höherer Reinheit erhalten werden. Die Zurückdrängung unerwünschter Nebenreaktionen bringt gleichzeitig eine Ausbeuteverbesserung mit sich. Dies wird beispielsweise deutlich durch die stark verkleinerte Menge an Reaktionsrückstand. Weiterhin erlaubt die erfindungsgemäße Anwendung der Harnstoff-Verbindungen die Umsetzung auch reaktionsträger Carbonsäuren, beispielsweise der halogenierten Essigsäuren.. Die erfindungsgemäß einsetzbaren Harnstoff-Verbindungen sind toxikologisch unbedenklich.

Überraschenderweise sind die erfindungsgemäß einsetzbaren Harnstoff-Verbindungen bereits in kleinen Mengen im Sinne der Reaktionsbeschleunigung wirksam, obwohl beim Vorhandensein von mindestens einem freien Wasserstoffatom in den erfindungsgemäß einsetzbaren Harnstoff-Verbindungen anzunehmen war, daß diese Harnstoff-Verbindung durch Reaktion mit dem Thionylhalogenid bereits zu Beginn der Reaktion irreversibel aufgebraucht würde.

Carbonsäurehalogenide sind dem Fachmann bekannte Substanzen, die beispielsweise für Acylierungen der verschiedensten Art eingesetzt werden können.

## Beispiele 1 bis 5 (vgl. Tabelle 1)

In einem 1 l-Dreihalskolben mit Rührer, Thermometer, Rückflußkühler und Blasenzähler fügt man zu 189,5 g (2,0 Mol) technisch reiner Monochloressigsäure den in der Tabelle 1 angegebenen Katalysator und bei Raumtemperatur 262 g (3,0 Mol) Thionylchlorid bei. Unter Rühren steigert man die Temperatur nach folgendem Plan: Nach 30 Min. sind 25°C zu erreichen, nach 60 Min. 33°C nach 90 Min. 39°C, nach 120 Min. 45°C, nach 180 Min. 55°C, nach 240 Min. 65°C, nach 300 Min. 76°C, nach 360 Min. 87°C und nach 390 Min. 90°C. Diese letzte Temperatur wird mit ±1,5°C Genauigkeit bis zum Ende der Reaktion beibehalten, was am Ende der Gasentwicklung zu erkennen ist. Durch die Einhaltung des

angegebenen Temperaturprogramms in allen zusammenhängenden Versuchen ist deren Vergleichbarkeit möglich.

Dann wird unverbrauchtes Thionylchlorid bis 76°C Übergangstemperatur abgetrennt und schließlich der Rückstand unter vermindertem Druck abdestilliert (Kp. 52–53,2°C/180 mm). Der Vorlauf enthält neben $SOCl_2$ bereits etwas Reaktionsprodukt. Mit Hilfe bekannter analytischer Verfahren (z. B. IR-spektroskopisch oder durch Umsetzung mit Di-n-butylamin) läßt sich der Anteil an Chloracetylchlorid bestimmen. Der Destillationsrückstand enthält unumgesetzte Chloressigsäure und/oder deren Anhydrid. (Angaben über Ausbeute oder Rückstandsmenge beziehen sich auf den Einsatz).

a) Vergleichsversuche (Beispiele 1 und 2)

Behandelt man wie oben beschrieben 189,5 g Chloressigsäure mit 262 g $SOCl_2$ ohne Katalysator, dann kommt die Umsetzung nach ca. 11 h zum Stillstand. Nach Destillation verbleiben 5,4 g Rückstand (2,9% des Einsatzes). Die Ausbeute an Chloracetylchlorid beträgt einschließlich des im Vorlauf enthaltenen Anteiles 96,5% der theoretischen Ausbeute.

Die gleichen Mengen an Chloressigsäure und $SOCl_2$ erfordern in Gegenwart von 0,146 g (0,1 Mol-%) Dimethylformamid (DMF) 7,2 Stunden bis zum Ende der Gasentwicklung; als Destillationsrückstand verbleiben 6,4 g (3,4% des Einsatzes; die Gesamtausbeute an Chloracetylchlorid beträgt 97,5% der theoretischen Ausbeute).

b) Erfindungsgemäße Umsetzungen (Beispiele 3 bis 5)

Behandelt man wie oben beschrieben 189,5 g Chloressigsäure mit 262 g $SOCl_2$, jeweils in Gegenwart von erfindungsgemäßen Katalysatoren, dann erhält man nach der beschriebenen Aufarbeitung die Ergebnisse der Tabelle 1:

Tabelle 1

| Beispiel Nr. | Katalysator | Menge Katal. [Mol.-%] | Reakt.-zeit [h] | Rückstand [g] | Rückstand [%] | Ausbeute [g] | Ausbeute [% d. Th.] |
|---|---|---|---|---|---|---|---|
| 1 | – | – | 11,0 | 5,4 | 2,9 | 218 | 96,5 |
| 2 | DMF | 0,1 | 7,25 | 6,4 | 3,4 | 220 | 97,5 |
| 3 | Harnstoff | 0,1 | 7,0 | 2,9 | 1,5 | 222 | 98,0 |
| 4 | Harnstoff | 0,3 | 6,3 | 1,9 | 1,0 | 223 | 98,5 |
| 5 | Guanidinbicarbonat | 0,1 | 8,3 | 1,5 | 0,8 | 223 | 98,5 |

Beispiel 6

In einem 1 l-Dreihalskolben mit Rührer, Innenthermometer, Rückflußkühler und Blasenzähler gibt man bei Raumtemperatur 94,5 g (1,0 Mol) Monochloressigsäure, 136,9 g (1,15 Mol) Thionylchlorid und 60 mg Harnstoff (0,1 Mol-%) zusammen und erwärmt in einem 100°C warmen Ölbad.

Nach 1,5 h ist die Gasentwicklung praktisch beendet. Man rührt nach 6 h bei dieser Temperatur nach und destilliert, ohne einen Vorlauf abzunehmen. Man erhält 104,5 g Destillat, das laut gaschromatographischer Analyse 98,1% Chloracetylchlorid und 1,3% $SOCl_2$ enthält (entspricht einer Ausbeute von 90,8% der theoretischen Ausbeute) und 6,8 g Rückstand, der noch weiteres Chloracetylchlorid enthält (etwa 90 Gew.-% des Rückstandes und damit weitere 5,4% der theoretischen Ausbeute) und in einen weiteren Ansatz zurückgeführt werden kann. Die Gesamtausbeute beträgt 96,2% der theoretischen Ausbeute.

Auf diese Art erhaltenes Chloracetylchlorid ist sehr rein und fast farblos.

Beispiele 7 bis 10 (vgl. Tabelle 2)

Umsetzungen von Methacrylsäure zum Methacrylchlorid werden durch die erfindungsgemäßen Katalysatoren nicht nur beschleunigt, sondern führen zu Reaktionsprodukten höherer Reinheit und zu höheren Ausbeuten.

In eine Reaktionsapparatur wie in Beispiel 1 gibt man bei 10–15°C zu 316 g (2,65 Mol) $SOCl_2$ höchster Reinheit im Verlauf von 30 Min. 206,5 g (2,40 Mol) Methacrylsäure und erwärmt das Gemisch nach folgendem Plan:

Nach 30 Min. ist das Zutropfen beendet, nach 60 Min. sind 30°C erreicht, nach 90 Min. 42–43°C, nach 120 Min. 56–57°C, nach 150 Min. 67°C, nach 180 Min. 74°C und nach 210 Min. 80–81°C.

Diese Temperatur wird bis zur Beendigung der Gasabspaltung beibehalten, dann der Rückflußkühler gegen einen absteigenden Kühler ausgetauscht und der Überschuß an $SOCl_2$ zusammen mit gebildetem Säurechlorid bis 104°C/760 mm Hg Übergangstemperatur abdestilliert. Der nicht destillierbare Anteil wird als Rückstand bezeichnet, die Reinheit des Destillats wird einerseits durch Bestimmung von $SOCl_2$- und $SO_2$-Gehalt mittels IR-Analyse, andererseits durch Bromierung der Methacrylchlorid-Doppelbindung bestimmt.

a) Vergleichsversuche (Beispiele 7 und 8)

Führt man die Umsetzung mit 316 g Thionylchlorid und 206,5 g Methacrylsäure ohne Katalysator nach vorgegebenem Aufheizplan durch, ist die Reaktion nach 4 h 15 Min. beendet. Die Destillation ergibt 53,0 g Rückstand und ein Destillat, das weniger als 0,5% $SOCl_2$ und $SO_2$ und 91,0% Methacrylchlorid enthält (durch Bromierung bestimmt).

Führt man die gleiche Umsetzung in Gegenwart von 55 mg (0,03 Mol-%) DMF durch, ist die Gasentwicklung nach 3 h 50 Min. beendet. Nach der Destillation verbleiben 25,6 g Rückstand. Das Destillat enthält weniger als 0,5% $SOCl_2$ und $SO_2$ und 91,3% Methacrylchlorid.

b) Erfindungsgemäße Umsetzungen (Beispiele 9 und 10)

Führt man die Umsetzungen mit den gleichen Mengen der Ausgangsmaterialien in Gegenwart von 0,03 Mol-% der genannten erfindungsgemäßen Katalysatoren durch, die dem Thionylchlorid vor der Umsetzung zugefügt werden, dann erhält man Ergebnisse, die in Tabelle 2 aufgeführt sind.

Tabelle 2

| Beispiel Nr. | Katalysator | Menge [Mol.-%] | Reaktionszeit [Min.] | Rückstand [q] | Gehalt des Destillates [%] |
|---|---|---|---|---|---|
| 7 | – | – | 255 | 53,0 | 91,0 |
| 8 | DMF | 0,03 | 230 | 25,6 | 91,3 |
| 9 | Harnstoff | 0,03 | 205 | – | 96,0 |
| 10 | Guanidinbicarbonat | 0,03 | 200 | – | 95,8 |

Beispiele 11 bis 19 (Vgl. Tabelle 3)

Der Vorteil der erfindungsgemäßen Katalysatoren bezüglich Reaktionsgeschwindigkeit und Ausbeute läßt sich auch an aromatischen Carbonsäuren nachweisen.

In einer 1 l-Rührapparatur wie in Beispiel 1 beschrieben werden bei Raumtemperatur 156,5 g (1,0 Mol) p-Chlorbenzoesäure mit 178,5 g (1,5 Mol) Thionylchlorid zusammengegeben, gegebenenfalls nach Zugabe des Katalysators zur Chlorbenzoesäure. Man erwärmt die Mischung nach folgendem Plan:

Im Verlauf von 15 Min. bis 35°C, von 30 Min. bis 52°C, von 45 Min. bis 65°C, von 60 Min. bis 70°C, von 90 Min. bis 80°C, von 180 Min. bis auf 90°C. Diese Temperatur wird bis zum Ende der Gasabspaltung beibehalten, sodann überschüssiges $SOCl_2$ bei 90°C unter leichtem Unterdruck abdestilliert und schließlich bei 103,5–105°C/15 mm Hg das 4-Chlorbenzoylchlorid fraktioniert.

Der nicht umgesetzte Rückstand wird in Gew.-% (bezogen auf eingesetzte 4-Chlorbenzoesäure) angegeben.

a) Vergleichsversuche (Beispiele 11 und 12)

Die unkatalysierte Umsetzung erfordert ca. 860 Min. bis zum Ende der Gasabspaltung, liefert 165,8 g (94,6% der theoretischen Ausbeute) an reinem 4-Chlorbenzoylchlorid und 8,8 g (5,5% des Einsatzes) an unreagiertem Einsatzmaterial.
Bei Anwendung von 0,073 g (0,1 Mol-%) DMF als Katalysator ist die Umsetzung in 295 Min. beendet und liefert 2,26 g Rückstand (1,45% der eingesetzten p-Chlorbenzoesäure) und 168,0 g (96,5% der theoretischen Ausbeute) an 4-Chlorbenzoylchlorid.

b) Erfindungsgemäße Umsetzung (Beispiele 13 bis 19)

Bei Verwendung der gleichen Ausgangsmaterialien und unter den gleichen Umsetzungsbedingungen, aber in Gegenwart der angegebenen Mengen der erfindungsgemäßen Katalysatoren, wurden die Ergebnisse der Tabelle 3 erhalten.

Tabelle 3

| Beispiel Nr. | Katalysator | Menge [Mol.-%] | Reaktionszeit [Min.] | Ausbeute [%] | Rückstand [%] |
|---|---|---|---|---|---|
| 11 | – | – | 860 | 94,6 | 5,5 |
| 12 | DMF | 0,1 | 295 | 96,5 | 1,45 |
| 13 | Harnstoff | 0,1 | 240 | 96,3 | 1,3 |
| 14 | Harnstoff | 0,3 | 170 | 97,3 | 1,7 |
| 15 | Harnstoff | 0,6 | 165 | 96,7 | 1,6 |
| 16 | N,N'-Dimethylharnstoff | 0,1 | 220 | 96,6 | 1,2 |
| 17 | N,N'-Diphenylharnstoff | 0,1 | 340 | 98,1 | 1,8 |
| 18 | N-Methylthioharnstoff | 0,1 | 500 | 94,8 | 1,7 |
| 19 | Guanidinbicarbonat | 0,1 | 290 | 98,2 | 1,8 |

Dabei wurde bei Verwendung von N-Methylthioharnstoff ein nur gering verfärbtes Produkt erhalten, was besonders wertvoll ist, wenn auf destillative Reinigung der Produkte verzichtet werden soll.

Beispiel 20

156,5 g (1,0 Mol) 2-Chlorbenzoesäure, 155 g (1,3 Mol) Thionylchlorid und 0,06 g (0,10 Mol-%) Harnstoff werden zusammengegeben und nach folgendem Plan erwärmt:
In 30 Min. bis auf 35° C, in 60 Min. bis auf 50° C, in 90 Min. bis auf 62° C, in 120 Min. bis auf 75° C und dann nach insgesamt 150 Min. bis auf 90° C; nach insgesamt 210 Min. ist die Gasentwicklung beendet. Beim Destillieren erhält man 171,8 g (98,2% der theoretischen Ausbeute) an 2-Chlorbenzoylchlorid und 1,88 g (1,2%) an Rückstand. Das erhaltene Produkt ist besonders rein (99–100% ermittelt durch Anilintitration); sein Erstarrungspunkt liegt bei –3,70° C.

### Beispiele 21 bis 25 (vgl. Tabelle 4)

In einer 1 l-Rührapparatur wie in Beispiel 1 werden bei 15–20°C 250,5 g (1,5 Mol) 2-Nitrobenzoesäure mit 268 g (2,25 Mol) $SOCl_2$ und gegebenenfalls mit Katalysator versetzt und nach folgendem Plan erwärmt:

Nach 30 Min. sind 30°C zu erreichen, nach 60 Min. 33°C, nach 90 Min. 35°C, nach 120 Min. 40°C, nach 140 Min. 45°C, nach 160 Min. 55°C, nach 180 Min. 65°C, nach 200 Min. 75°C, nach 220 Min. 85°C und nach 4 h 90°C.

Diese Temperatur wird bis zum Ende der Gasentwicklung beibehalten, dann wird bei 90°C/200 mm Hg überschüssiges $SOCl_2$ abdestilliert und das 2-Nitrobenzoylchlorid bei 121–122°C/0,4 mm Hg fraktioniert. Das hellgelbe Destillat ist wenigstens 99,8%ig (Anilintitration). Die Ergebnisse sind in Tabelle 4 zusammengefaßt (Vergleichsbeispiele 21 und 22; Beispiele 23 bis 25).

### Beispiel 26

In einer Apparatur, wie in Beispiel 6 beschrieben, gibt man bei Raumtemperatur 94,5 g (1,0 Mol) Monochloressigsäure, 136,9 g (1,15 Mol) Thionylchlorid und 1,4 g N-Chloracetyl-harnstoff zusammen und erwärmt in einem 100°C warmen Ölbad. Nach 1,5 Stunden ist die Gasentwicklung beendet. Man rührt noch 6 Stunden bei dieser Temperatur nach und destilliert, ohne einen Vorlauf abzunehmen. Man erhält 96,9 g Destillat, das laut gaschromatographischer Analyse 99,4 Gew.-% Chloracetylchlorid enthält (das entspricht einer Ausbeute von 85,7% der theoretischen Ausbeute), ferner 8,5 g Rückstand.

Dieser Rückstand wird zusammen mit 1 Mol Monochloressigsäure und 137 g $SOCl_2$ wie beschrieben umgesetzt. Man erhält bei der Destillation 107,8 g 99,5 gew.-%iges Chloracetylchlorid (entspricht 95,0% der theoretischen Ausbeute) sowie 15,7 g Rückstand, der beim Erkalten erstarrt.

Die Gesamtausbeute beider Reaktionsläufe beträgt 90,4% der theoretischen Ausbeute.

Tabelle 4

| Beispiel Nr. | Katalysator | Menge [Mol.-%] | Reaktionszeit [Min.] | Rückstand [%] | Ausbeute [%] |
|---|---|---|---|---|---|
| 21 | – | – | 500 | 2,35 | 97,0 |
| 22 | DMF | 0,1 | 310 | 1,8 | 98,0 |
| 23 | Harnstoff | 0,1 | 300 | 2,0 | 97,4 |
| 24 | Harnstoff | 0,25 | 270 | 1,3 | 98,3 |
| 25 | N,N'-Dimethylharnstoff | 0,1 | 270 | 1,8 | 98,2 |

## Patentansprüche

1. Verfahren zur Herstellung von Carbonsäurehalogeniden durch Umsetzung einer Carbonsäure der Formel

$$R-COOH,$$

in der

R für Aryl oder $R^1R^2R^3C-$ steht,
worin $R^1$, $R^2$ und $R^3$ unabhängig voneinander Wasserstoff, Halogen, Alkyl oder Alkenyl bedeuten und worin weiterhin zwei der Reste $R^1$ bis $R^3$ gemeinsam eine Alkylengruppe oder eine über eine Doppelbindung gebundene Alkylidengruppe bedeuten können, wobei die Kohlenstoff enthaltenden Reste durch Carboxyl, eine Estergruppe, deren Alkoholkomponente 1 bis 4 Kohlenstoffatome enthält, $C_1-C_{10}$-Alkoxy, Aryloxy, Aralkoxy, eine $C_1-C_{10}$-Thioethergruppe, Niederalkyl, Cyano, Nitro oder Halogen substituiert sein können,

mit einem Thionylhalogenid der Formel

$$SOX_2,$$

in der X für Chlor oder Brom steht, in Gegenwart von Zusätzen, dadurch gekennzeichnet, daß man als Zusatz 0,01 bis 5 Mol-%, bezogen auf die Äquivalente der eingesetzten Carbonsäure, einer gegebenenfalls substituierten Harnstoff-Verbindungen der Formel

$$R^4NH-CY-NR^5R^6,$$

in der

Y doppelt gebundenen Sauerstoff, Schwefel oder gegebenenfalls substituierten Stickstoff, $R^7-N=$ bedeutet und
$R^4$, $R^5$, $R^6$ und $R^7$ unabhängig voneinander für Wasserstoff, Alkyl, Aralkyl, Aryl oder Acyl stehen, wobei weiterhin die zwei Reste $R^4$ und $R^5$, $R^5$ und $R^6$ oder $R^5$ und $R^7$ eine Alkylengruppe bilden können und wobei die Kohlenstoff enthaltenden Reste durch die obengenannten Substituenten substituiert sein können,

in freier Form oder in Form ihres Salzes, gegebenenfalls in Gegenwart eines inerten Lösungsmittels, einsetzt.

2. Verfahren nach Anspruch 1, dadurch gekennzeichnet, daß man 0,02 bis 2 Mol-% einer Harnstoff-Verbindung, bezogen auf die eingesetzten Carbonsäureäquivalente, einsetzt.

3. Verfahren nach Anspruch 1 und 2, dadurch gekennzeichnet, daß man Harnstoff einsetzt.

4. Verfahren nach Anspruch 1 bis 3, dadurch gekennzeichnet, daß bei einer Temperatur von 0 bis 150° C gearbeitet wird.

5. Verfahren nach Anspruch 1 bis 4, dadurch gekennzeichnet, daß bei Rückflußtemperatur des Reaktionsgemisches gearbeitet wird.

6. Verfahren nach Anspruch 1 bis 5, dadurch gekennzeichnet, daß man eine gegebenenfalls substituierte Harnstoff-Verbindung der Formel

$$R^{16}NH-CY^1-NHR^{17}$$

einsetzt, in der

$Y^1$ doppelt gebundenen Sauerstoff, doppelt gebundenen Schwefel oder gegebenenfalls substituierten doppelt gebundenen Stickstoff $R^{18}-N=$ bedeutet und
$R^{16}$, $R^{17}$ und $R^{18}$ unabhängig voneinander für Wasserstoff, $C_1-C_4$-Alkyl, Phenyl oder Acyl stehen.

7. Verfahren nach Anspruch 1 bis 6, dadurch gekennzeichnet, daß man 0,03 bis 1 Mol-% einer Harnstoff-Verbindung, bezogen auf die eingesetzten Carbonsäureäquivalente, einsetzt.

8. Verfahren nach Anspruch 1 bis 7, dadurch gekennzeichnet, daß das Thionylhalogenid in mindestens äquivalenter Menge pro freies Carboxyläquivalent eingesetzt wird.

**Claims**

1. Process for the preparation of carboxylic acid halides by reacting a carboxylic acid of the formula

$$R-COOH$$

in which

R represents aryl or $R^1R^2R^3C-$,

wherein

$R^1$, $R^2$ and $R^3$ independently of one another denote hydrogen, halogen, alkyl or alkenyl,

and wherein, furthermore,

two of the radicals $R^1$ to $R^3$ together can denote an alkylene group, or an alkylidene group which is bonded via a double bond, it being possible for the radicals containing carbon to be substituted by carboxyl, an ester group, the alcohol component of which contains 1 to 4 carbon atoms, $C_1-C_{10}$-alkoxy, aryloxy, aralkoxy, a $C_1-C_{10}$-thioether group, lower alkyl, cyano, nitro or halogen,

with a thionyl halide of the formula

$SOX_2$

in which X represents chlorine or bromine,
in the presence of additives, characterised in that 0.01 to 5 mol%, relative to the equivalents of the carboxylic acid employed, of an optionally substituted urea compound of the formula

$R^4NH-CY-NR^5R^6$

in which

Y denotes double-bonded oxygen, sulphur or optionally substituted nitrogen $R^7-N=$ and $R^4$, $R^5$, $R^6$ and $R^7$ independently of one another represent hydrogen, alkyl, aralkyl, aryl or acyl,

wherein, furthermore,

the two radicals $R^4$ and $R^5$, $R^5$ and $R^6$ or $R^5$ and $R^7$ can form an alkylene group

and wherein

the radicals containing carbon can be substituted by the abovementioned substituents,

in the free form or in the form of its salt, if appropriate in the presence of an inert solvent, are employed as the additive.

2. Process according to Claim 1, characterised in that 0.02 to 2 mol% of a urea compound, relative to the carboxylic acid equivalents employed, is used.

3. Process according to Claim 1 and 2, characterised in that urea is employed.

4. Process according to Claim 1 to 3, characterised in that the reaction is carried out at a temperature of 0 to 150° C.

5. Process according to Claim 1 to 4, characterised in that the reaction is carried out at the reflux temperature of the reaction mixture.

6. Process according to Claim 1 to 5, characterised in that an optionally substituted urea compound of the formula

$R^{16}NH-CY^1-NHR^{17}$

is used, in which

$Y^1$ denotes double-bondes oxygen, double-bonded sulphur or optionally substituted double-bonded nitrogen $R^{18}-N=$ and
$R^{16}$, $R^{17}$ and $R^{18}$ independently of one another represent hydrogen, $C_1-C_4$-alkyl, phenyl or acyl.

7. Process according to Claim 1 to 6, characterised in that 0.03 to 1 mol% of a urea compound, relative to the carboxylic acid equivalents employed, is used.

8. Process according to Claim 1 to 7, characterised in that the thionyl halide is used in at least an equivalent amount per free carboxyl equivalent.

**Revendications**

1. Procédé de préparation d'halogénures d'acides carboxyliques par réaction d'un acide carboxylique de formule

$R-COOH$

dans laquelle

R représente un groupe aryle ou $R^1R^2R^3C-$,

$R^1$, $R^2$ et $R^3$ représentent chacun, indépendamment les uns des autres, l'hydrogène, un halogène, un groupe alkyle ou alcényle et en outre deux des restes $R^1$ à $R^3$ peuvent former ensemble un groupe alkylène ou un groupe alkylidène relié par l'intermédiaire d'une double liaison, les restes contenant du carbone pouvant être substitués par des groupes carboxyle, un groupe ester dont le composant alcool contient de 1 à 4 atomes de carbone, des groupes alcoxy en $C_1-C_{10}$, aryloxy, aralcoxy, un groupe

thioéther en $C_1-C_{10}$, alkyle inférieur, cyano, nitro ou des halogènes, avec un halogénure de thionyle de formule

$$SOX_2$$

dans laquelle X représente le chlore ou le brome, en présence d'additifs, caractérisé en ce que l'on utilise en tant qu'additif 0,01 à 5 moles %, par rapport aux équivalents de l'acide carboxylique mis en oeuvre, d'un dérivé de l'urée éventuellement substitué répondant à la formule

$$R^4NH-CY-NR^5R^6$$

dans laquelle
Y représente l'oxygène, le soufre et l'azote éventuellement substitué relié par une double liaison, $R^7-N=$ et
$R^4$, $R^5$, $R^6$ et $R^7$ représentent chacun, indépendamment les uns des autres, l'hydrogène, un groupe alkyle, aralkyle, aryle ou acyle, et en outre les deux restes $R^4$ et $R^5$, $R^5$ et $R^6$ ou $R^5$ et $R^7$ peuvent former un groupe alkylène, les restes contenant du carbone pouvant être substitués par les substituants mentionnés ci-dessus,
à l'état libre ou l'état de sel, éventuellement en présence d'un solvant inerte.

2. Procédé selon la revendication 1, caractérisé en ce que l'on utilise de 0,02 à 2 moles % d'un dèrivé de l'urée par rapport aux èquivalents d'acide carboxylique mis en oeuvre.

3. Procédé selon les revendications 1 et 2, caractérisé en ce que l'on utilise l'urée.

4. Procédé selon les revendications 1 à 3, caractérisé en ce que l'on opère à une température de 0 à 150° C.

5. Procédé selon les revendications 1 à 4, caractérisé en ce que l'on opère à la température de reflux du mélange de réaction.

6. Procédé selon les revendications 1 à 5, caractérisé en ce que l'on utilise un dérivé de l'urée éventuellement substitué de formule

$$R^{16}NH-CY^1-NHR^{17}$$

dans laquelle
$Y^1$ représente l'oxygène relié par une double liaison, le soufre relié par une double liaison ou l'azote éventuellement substitué relié par une double liaison $R^{18}-N=$, et
$R^{16}$, $R^{17}$ et $R^{18}$ représentent chacun, indépendamment les uns des autres, l'hydrogène, un groupe alkyle en $C_1-C_4$, phényle ou acyle.

7. Procédé selon les revendications 1 à 6, caractérisé en ce que l'on utilise de 0,03 à 1 mole % d'un dérivé de l'urée par rapport aux équivalents d'acide carboxylique mis en oeuvre.

8. Procédé selon les revendications 1 à 7, caractérisé en ce que l'on utilise l'halogénure de thionyle en quantité au moins équivalente par équivalent de carboxyle libre.